# EUROPÄISCHE PATENTSCHRIFT

(11) **EP 0 441 923 B1**
(45) Veröffentlichungstag und Bekanntmachung des Hinweises auf die Patenterteilung: **25.10.1995**
(21) Anmeldenummer: 90912138.6
(22) Anmeldetag: 28.08.1990
(51) Int. Cl.: A61C 7/12

(54) **VORRICHTUNG ZUR TEMPORÄREN ZAHNSCHIENUNG**
DEVICE FOR TEMPORARILY SUPPORTING TEETH
DISPOSITIF POUR L'ECLISSAGE DE DENTS

(30) Priorität: 29.08.1989 CH 3131/89
(43) Veröffentlichungstag der Anmeldung: 21.08.1991
(73) Patentinhaber: Baer, Hans, Dr., CH-8006 Zürich (CH); Hirsbrunner, Eduard, Dr., CH-8006 Zürich (CH)
(72) Erfinder: Baer, Hans, Dr., CH-8006 Zürich (CH); Hirsbrunner, Eduard, Dr., CH-8006 Zürich (CH)
(74) Vertreter: Ritscher, Thomas, Dr.
(86) Internationale Anmeldenummer: CH9000202
(87) Internationale Veröffentlichungsnummer: WO9103212

(56) Entgegenhaltungen:
- US-A- 3 505 736
- US-A- 4 107 844

## Beschreibung

Die vorliegende Erfindung betrifft eine Vorrichtung, wie sie im Oberbegriff des unabhängigen Patentanspruchs 1 definiert ist.

Verletzungen im Zahn- und Kieferbereich führen in vielen Fällen zur Lockerung von Zähnen, die meistens noch gerettet werden können, vorausgesetzt, dass die erforderliche Notfallbehandlung rasch erfolgen kann. Im Rahmen einer derartigen Notfallbehandlung werden die traumatisierten Zähne reponiert und müssen dann anschliessend möglichst umgehend durch Schienung an den nicht luxierten Nachbarzähnen vorübergehend fixiert werden. Die sofortige Reposition und Schienung ist eine absolut vordringliche Voraussetzung einer erfolgreichen Behandlung wegen der raschen Degeneration des Restparodontes sowie der Bildung von Blutgerinnseln im Alveolenbereich, die das Repositionieren des luxierten Zahnes behindern. Auch besteht das Risiko, dass sich ein zunächst teilweise luxierter Zahn nach einer gewissen Zeit löst und in die Luftröhre gelangt.

Schienungen im Zahnbereich können ferner zur Erzielung temporärer kieferchirurgischer Fixationen, und zwar intramandibulär, intramaxillär oder intermaxillär, erforderlich sein.

Gemäss einer bekannten, zur Zeit angewandten Technik wird der luxierte und reponierte Zahn mit seinen Nachbarzähnen mittels eines Metalldrahtes verbunden, der auf der Frontfläche der Zähne nach einer ätzenden Vorbehandlung durch ein Kompositmaterial fixiert wird. Die Anwendung dieser bekannten Methode erfordert eine spezielle Beherrschung von Material und Technik und kann so nur von einem Zahnarzt, nicht aber durch den in solchen Fällen meist zunächst zugezogenen bzw. verfügbaren Notfall- oder Hausarzt ausgeführt werden.

Für die langfristige Nachbehandlung kieferorthopädischer Fälle ist es bekannt, starre, metallische Halteelemente, sogenannte "brackets", auf die Zahnoberfläche aufzukleben. Sämtliche dieser Halteelemente sind durch einen lose durch dieselben hindurchgeführten Metalldraht miteinander verbunden. Durch Spannen und erforderlichenfalls periodisches Nachspannen des Drahtes werden über die Halteelemente, welche in diesem Falle eine kraftübertragende Funktion haben, richtende Spannkräfte auf die zu behandelnden Zähne übertragen. Auch diese Methode kann nur von dem mit ihr vertrauten Zahnarzt angewandt werden; ausserdem erschwert die Starrheit der Halteelemente deren exakte Positionierung.

Es ist auch schon versucht worden, eine Zahnschienung nur mit Kunststoff, das heisst ohne drahtförmiges Bindeglied, zu bewerkstelligen. Diese Methode konnte sich jedoch nicht durchsetzen, da es schwierig ist, den Kunststoff exakt zu formen und er andererseits den im Zahnbereich auftretenden Kräften oft nicht standhält. Auch hat sich die Entfernung des Kunststoffes als schwierig erwiesen.

Da das Kompositmaterial nach einer gewissen Zeit wieder entfernt werden muss, ist es von Bedeutung, dass von vornherein eine möglichst geringe Menge dieses Materials aufgetragen wird, die Klebefläche also möglichst exakt begrenzt wird, während andererseits aber doch das drahtförmige Bindeglied an der jeweiligen Befestigungsstelle sicher von Komposit umschlossen sein soll und die Bildung von Hohlstellen, insbesondere zwischen Draht und Zahnoberfläche, vermieden werden muss.

Es ist die Aufgabe der vorliegenden Erfindung, eine Vorrichtung zur temporären passiven Schienung von Zähnen vorzuschlagen, die nicht nur von Zahnärzten, sondern insbesondere auch vom Hausarzt, dem Notfallarzt, dem Kieferchirurgen und gegebenenfalls auch von medizinischem Hilfspersonal risikolos angewandt werden kann.

Die neue Vorrichtung soll einerseits die Anbringung des das drahtförmige Bindeglied an der Zahnoberfläche sichernden Komposits auf die effektiv notwendige Kompositmenge beschränken und es andererseits gestatten, die Lage der Befestigungsstelle an der Zahnoberfläche exakt zu wählen.

Die Erfindung ist im kennzeichnenden Teil des unabhängigen Patentanspruchs 1 definiert, wobei sich bevorzugte Ausführungsbeispiele aus den abhängigen Ansprüchen ergeben.

Nachstehend werden einige Ausführungsbeispiele der erfindungsgemässen Vorrichtung unter Bezugnahme auf die beiliegende Zeichnung beschrieben.
Fig. 1 veranschaulicht zunächst die Anwendung der erfindungsgemässen Vorrichtung an den Aussenflächen der maxilla- und mandibulaseitigen Zähne,
Fig. 2 zeigt eine erste Ausführungsform der erfindungsgemässen Vorrichtung,
Fig. 3 zeigt die Applizierung der Vorrichtung an der Aussenfläche einer maxillaseitigen Zahnreihe,
Fig. 4 ist eine Frontansicht eines Zahnbereichs, welcher auf den Zahnaussenflächen sowohl maxilla- als auch mandibulaseitig geschient wurde, wobei beide Kieferpartien intermaxillär gekoppelt sind,
Fig. 5 zeigt eine Anwendung auf den Zahninnenflächen lingual und palatinal,
Fig. 6 zeigt einige mögliche Formen der Kompositträger und die
Fig. 7 bis 11 veranschaulichen Anwendungsbeispiele an den oralen und/oder fazialen Oberflächen der maxilla- und/oder mandibulaseitigen Zahnpartien.

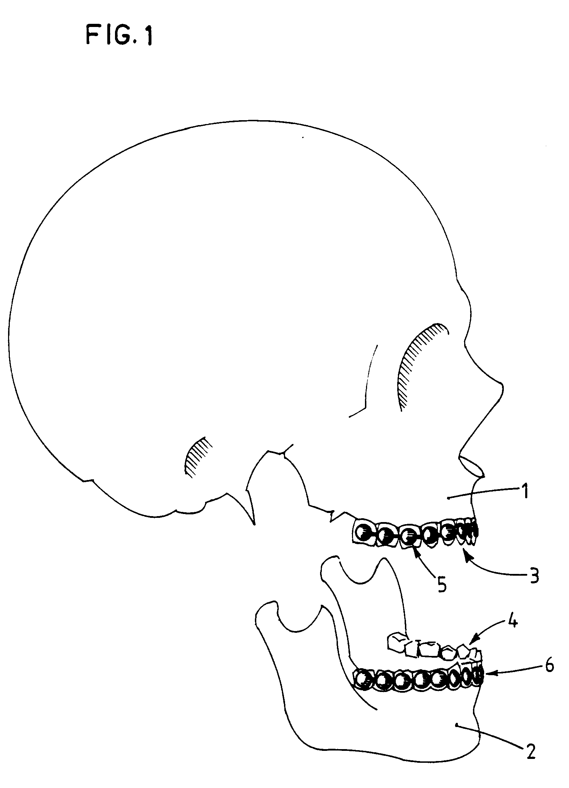

Fig. 1 zeigt die Zahnreihen von Oberkiefer (Maxilla)1 und Unterkiefer (Mandibula)2 an welchen anschauungshalber eine Ausführungsform der erfindungsgemässen Vorrichtung dargestellt ist. An den Aussenflächen der mit 3 bzw. 4 bezeichneten Zähne sind Schienungen 5 bzw. 6 angebracht, wie sie beispielsweise erforderlich sein können, um einen traumatisierten Zahn an seinen Nachbarzähnen temporär zu fixieren oder um eine ganze Kieferpartie (beispielsweise bei Kieferbruch) vorübergehend ruhig zu stellen.

Zur exakten Abgrenzung der vorliegenden Erfindung sei zunächst darauf hingewiesen, dass dieselbe weder zur aktiven Schienung noch zur langfristigen orthopädischen Behandlung von Zahnstellungs- bzw. Kieferanomalien gedacht ist. Der Anwendungsbereich der erfindungsgemässen Vorrichtung liegt ausschliesslich in der temporären passiven Fixation von Zähnen, Zahnreihen oder Kieferpartien, wobei in erster Linie an die unmittelbar nach Unfällen bzw. chirurgischen Eingriffen zu ergreifenden Massnahmen gedacht ist, die bisher ohne Beizug eines sachkundigen Zahnarztes oder Kieferchirurgen nicht ausgeführt werden konnten, die aber dank der erfindungsgemässen Lehre nun auch von dem nach einem Unfall oder einer Operation anwesenden Betreuungspersonal (Hausarzt, Notfallarzt, Chirurg, Pflegepersonal) dank der einfachen Applikationsmöglichkeit ohne weiteres durchführbar sind.

Es wird demnach zwecks Anwendung der erfindungsgemässen Vorrichtung vorausgesetzt, dass eventuell traumatisch dislozierte Zähne vorab reponiert wurden und demnach nur noch eine vorübergehende Fixierung erforderlich ist.

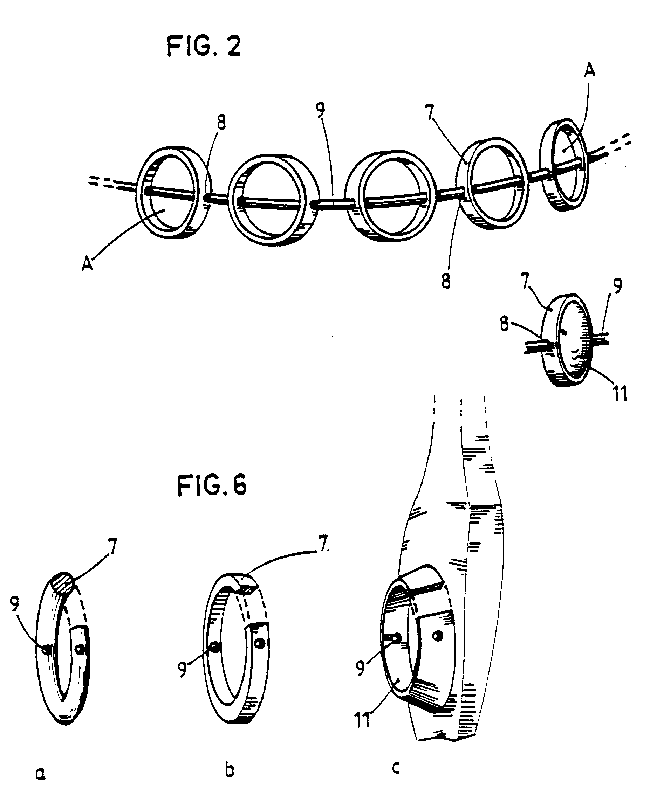

Zu diesem Zwecke wird eine vorgefertigte Schienungsvorrichtung verwendet, wie sie beispielsweise in Fig. 2 dargestellt ist. Die Vorrichtung weist eine Reihe Kompositträger 7 auf, deren einander gegenüberliegende Wandteile jeweils eine Durchgangsbohrung 8 aufweisen, durch welche ein Draht (Metall oder Kunststoff) 9 so hindurchgezogen ist, dass sich die Kompositträger 7 längs des Drahtes verschieben lassen.

Die Kompositträger 7 bestehen aus einem flexiblen Kunststoff und sind beispielsweise Abschnitte eines extrudierten Kunststoffschlauches, so dass sie sich durch Fingerdruck plastisch verformen lassen. Als Ausgangsmaterial kommt für die Kompositträger 7 beispielsweise Polymethylmethacrylat in Frage. Der Draht 9 kann entweder aus rostfreiem Stahl (Metall) oder einem geeigneten Kunststoff bestehen.

Diese in Fig. 2 schematisch dargestellte Schienungsvorrichtung wird nun auf die zu verbindende Zahnpartie aufgebracht, nachdem die vorgesehene Befestigungsstelle mit einem Aetzmittel, im allgemeinen 40%iger Phosphorsäure, vorbehandelt wurde. Zu diesem Zwecke wird das Schienungsmaterial, das beispielsweise als Meterware vorrätig gehalten werden kann, auf die erforderliche Länge abgeschnitten und der Krümmung der betreffenden Zahnreihe annähernd angepasst. Nachdem die Kompositträger auf die vorgesehenen Befestigungsstellen aufgelegt wurden, lassen sich dieselben dank ihrer Verschiebbarkeit auf dem Draht 9 und ihrer elastischen Verformbarkeit optimal plazieren. In dieser Behandlungsphase liegen somit sämtliche Kompositträger 7 auf den Befestigungsstellen auf, während der Draht 9 sämtliche Kompositträger 7 durchdringt, die Zahnoberflächen jedoch nicht berührt, sondern im Abstand vor denselben geführt ist. Nun werden die von den Kompositträgern 7 umgrenzten Ausnehmungen A mit Kompositmaterial (K) ausgefüllt und überschüssiges Kompositmaterial abgestreift. Damit ist auf einfache Weise eine angenehm zu tragende Fixierung erzielt, die sich - falls das erforderliche Zubehör verfügbar ist - auch von Nichtdentisten in kürzester Frist anbringen lässt.

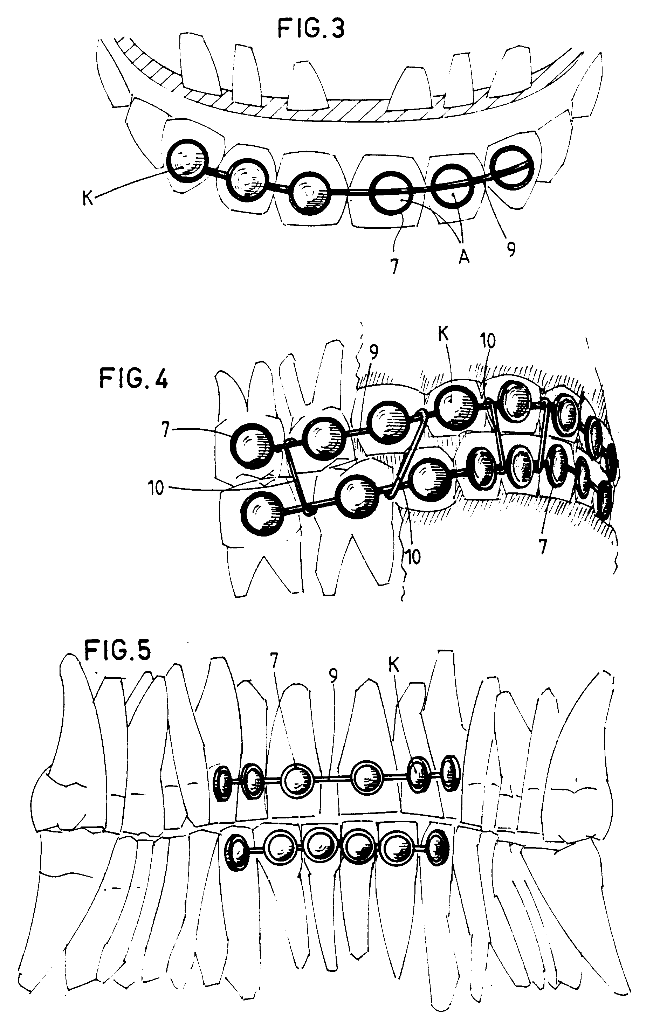

In Fig. 3 ist die Schienung am Beispiel einer maxillaseitigen Zahnfixation nochmals im Ausschnitt und in etwas grösserem Massstab dargestellt. Dabei ist die linke Hälfte der Schienung mit den dort befindlichen drei Kompositträgern 7 bereits mittels Kompositmaterial K an den angrenzenden Zahnoberflächen befestigt, während die Ausnehmungen A der restlichen drei Kompositträger 7 noch mit Kompositmaterial zu versehen sind.

Sollte sich die Fixationsmethode in Einzelfällen als unzureichend erweisen, so kann sie selbstverständlich mit bekannten Fixationsmitteln, beispielsweise einer einen einzelnen Zahn umgreifenden zusätzlichen Drahtligatur, kombiniert werden.

Eine weitere Anwendung zeigt Fig. 4. Hier mag beispielsweise das Erfordernis vorliegen, Maxilla und Mandibula im Hinblick auf die Heilung eines Kieferbruches vorübergehend aneinander zu fixieren, um die Bruchstelle dadurch während der ersten Periode der Heilungszeit ruhig zu stellen. Es sind in diesem Falle sowohl die Aussenflächen der oberen (maxillaseitigen) als auch die der unteren (mandibulaseitigen) Zähne mit einer Schienung versehen. Zur gegenseitigen, "intermaxillären" Verbindung der beiden Kiefer sind die beiden Drähte 9 mittels Verbindungsorganen 10 aneinandergekoppelt. Diese Verbindungsorgane 10 können beispielsweise metallische, C-förmige, plastisch verbiegbare Klammern, selbstfixierende Plastikschlaufen oder auch einfache Drahtligaturen sein.

Die Anbringung der beschriebenen Schiene an den Zahninnenflächen (lingual und palatinal) zeigt schematisch Fig. 5. Diese Art der Schienung hat unter anderem den Vorteil, dass sie von aussen unsichtbar ist. In Verbindung mit Schienen an der Zahnaussenfläche ist eine Erhöhung der Schienungsstabilität intramaxillär, intramandibulär sowie auch intermaxillär erreichbar.

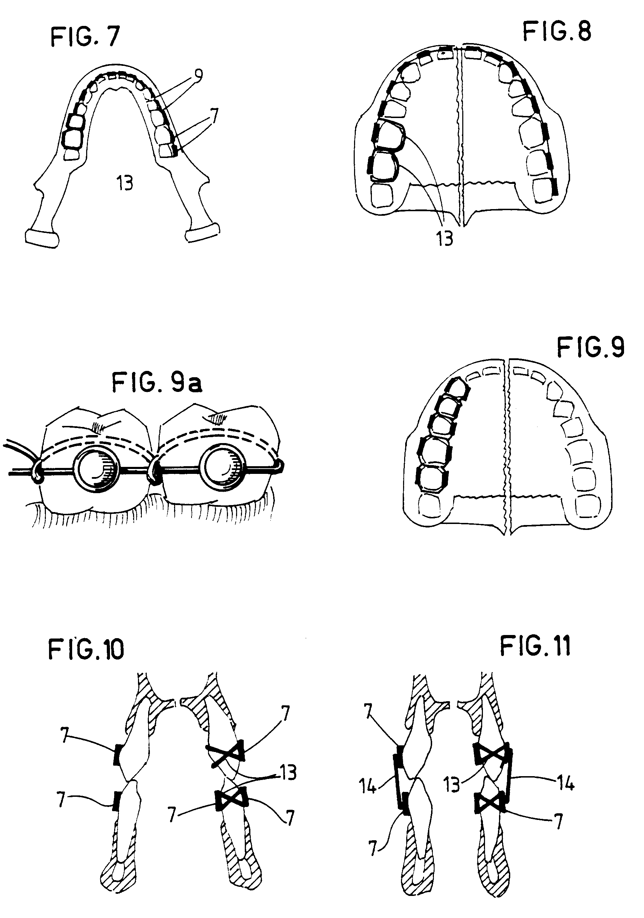

Das im Rahmen des vorliegenden Erfindungsgedankens ausschlaggebende Element ist der Kompositträger 7, der es gestattet, die einfache Anwendung der Vorrichtung mit gutem Tragkomfort und ausgezeichneter Stabilität zu verbinden. Drei mögliche Querschnittsformen von Kompositträgern 7, das heisst ein Ringwulst a von kreisförmigem Querschnitt, ein zylindrischer Abschnitt b und eine kegelstumpfförmige Variante c, sind in Fig. 6 veranschaulicht, wobei nur bei der kegelstumpfförmigen Variante c der angrenzende Zahn Z angedeutet ist. Hier ist ersichtlich, wie das Kompositmaterial 11 den vom Kompositträger 7 umgrenzten Hohlraum ausfüllt, dabei den von der Zahnoberfläche im Abstand befindlichen Draht 9 allseitig umschliesst und sowohl den Kompositträger 7 als auch den Draht 9 sicher mit der Zahnoberfläche verbindet. Die Kompositträger 7 können je nach Bedarf rund, oval oder auch polygonal sein. Die beiden Durchgangslöcher 8 (Fig. 2) für den Draht 9 müssen nicht unbedingt diametral einander gegenüberliegen, sondern können in Sonderfällen auch gegeneinander versetzt sein.

Die Hauptanwendungen der beschriebenen Schienung sind in den Fig. 7 bis 11 nochmals schematisch dargestellt. Es zeigen
Fig. 7 eine intramandibuläre buccale Schienung, mit an zwei Zähnen angebrachter Drahtligatur 13,
Fig. 8 eine intramaxilläre buccale Schienung, ebenfalls mit zusätzlicher, zweifacher Sicherung durch Drahtligatur 13,
Fig. 9 eine beidseitige, das heisst buccal/linguale Schienung, wobei die Drähte der beiden Schienungen mittels Drahtligaturen 12 verbunden sind,
Fig. 10 eine intramaxilläre und intramandibuläre Fixation, die durch lokale Drahtligaturen 13 gesichert ist und
Fig. 11 eine intermaxilläre Fixation, bei welcher die Schienendrähte der beiden Kiefer mittels plastisch verbiegbarer Metallklammern, selbstfixierender Kunststoffschlaufen oder Drahtligaturen 14 aneinander gesichert sind.

Es versteht sich, dass weitere Ausformungen der beschriebenen Kompositträger im Bereich des fachmännischen Wissens und Könnens liegen.

## Patentansprüche

1. Vorrichtung zur temporären passiven Fixierung von Zähnen, Zahnreihen oder Kieferpartien, bei Zahnluxation und bei kieferchirurgischen Eingriffen, wobei auf der äusseren (fazialen) und/oder inneren (oralen) Zahnoberfläche mehrerer nebeneinanderliegender Zähne ein drahtförmiges Bindeglied verankert wird, dadurch gekennzeichnet, dass das drahtförmige Bindeglied (9) eine Reihe verschiebbar auf demselben angeordneter, aus einem flexiblen Kunststoff erstellter Kompositträger (7) aufweist, die dazu bestimmt sind, das aufzutragende Kompositmaterial (K) lokal zu begrenzen und die korrekte Fixierung des drahtförmigen Bindegliedes (9) auf der Zahnoberfläche zu gewährleisten, wobei jeder Kompositträger (7) eine zur Aufnahme und Begrenzung des auf die Zahnoberfläche aufzutragenden Kompositmaterials (K) dienende Ausnehmung (A) umgrenzt und das drahtförmige Bindeglied (9) die einander gegenüberliegenden Wandungen jedes Kompositträgers (7) so durchquert, dass es nach dessen Applizierung im Abstand von den Zahnoberflächen liegt und nach dem Auftragen des Kompositmaterials allseitig von demselben umschlossen ist.

2. Vorrichtung nach Anspruch 1, dadurch gekennzeichnet, dass die Kompositträger (7) an zwei praktisch einander gegenüberliegenden Seiten mindestens je ein Durchgangsloch (8) aufweisen, durch welche das drahtförmige Bindeglied (9) hindurchgeführt ist.

3. Vorrichtung nach einem der Ansprüche 1 oder 2, dadurch gekennzeichnet, dass die Kompositträger (7) eine ergonomische Form aufweisen.

4. Vorrichtung nach Anspruch 3, dadurch gekennzeichnet, dass die Kompositträger (7) hakenartige Anformungen (15) aufweisen.

5. Vorrichtung nach Anspruch 3, dadurch gekennzeichnet, dass die Kompositträger (7) Oeffnungen (16, 17) zum Füllen der Kompositträger (7) und zum Härten des Kompositmaterials aufweisen.

6. Vorrichtung nach einem der Ansprüche 1 bis 3, dadurch gekennzeichnet, dass das drahtförmige Bindeglied (9) aus rostfreiem Stahl oder einem elastisch verformbaren biokompatiblen Material und die Kompositträger (7) aus plastisch verformbarem Kunststoff bestehen.

## Claims

1. Device for the temporary passive fixation of teeth, teeth rows or jaw parts in the case of tooth dislocation and in the case of surgical interventions on jaws, whereby a wire-shaped connecting link is anchored on the outer (facial) and/or inner (oral) surfaces of several neighbouring teeth, characterized in that a row of composite carriers (7) made of a flexible synthetic material are movably arranged on said wire-shaped connecting link (9), which composite carriers are intended to locally limit the composite material (K) to be applied and to ensure correct fixation of the wire-shaped connecting link (9) on the tooth surface, whereby each composite carrier (7) bounds a recess (A) which is adapted to receive and limit spreading of said composite material to be applied to the tooth surface, and said wire-shaped connecting link (9) passes through each of the composite carrier walls lying oppositely to each other in such a way that after its affixation at a distance to the teeth surfaces and after application of said composite material it is surrounded on all sides by said composite material.

2. Device according to claim 1, characterized in that the composite carriers (7) comprise on each of two sides which lie substantially opposite one another, a through hole (8), through which the wire-shaped connecting link is guided.

3. Device according to either claim 1 or 2, characterized in that the composite carriers (7) have an ergonomic design.

4. Device according to claim 3, characterized in that the composite carriers (7) comprise hook-like appendages (15).

5. Device according to claim 3, characterized in that the composite carriers (7) comprise openings (16, 17) for filling the composite carriers (7) and for curing the composite material.

6. Device according to one of claims 1 to 3, characterized in that the wire-shaped connecting link (9) is made of a stainless steel or an elastically deformable biocompatible material, and the composite carriers (7) are made of a plastically deformable synthetic material.

## Revendications

1. Dispositif pour la fixation passive temporaire de dents, de rangées de dents ou de parties de mâchoire en cas de luxation dentaire et en cas d'interventions chirurgicales maxillaires, dans lequel un organe de liaison en forme de fil est ancré sur la surface dentaire extérieure (faciale) et/ou interne (orale) de plusieurs dents juxtaposées, caractérisé en ce que l'organe de liaison (9) en forme de fil comporte une série de supports composites (7) disposés de façon à pouvoir se déplacer sur l'organe et réalisés en une matière plastique flexible, supports qui sont destinés à limiter localement le matériau composite (K) à mettre en place et à garantir la fixation correcte de l'organe de liaison (9) en forme de fil sur la surface dentaire, chaque support composite (7) délimitant un évidement (A) servant à loger et à limiter le matériau composite (K) devant être appliqué sur la surface dentaire, et l'organe de liaison (9) en forme de fil traversant les parois, opposées entre elles, de chaque support composite (7) de telle façon que cet organe, après son application, soit situé à distance des surfaces dentaires et soit entouré de tous côtés par le matériau composite après l'application de ce dernier.

2. Dispositif selon la revendication 1, caractérisé en ce que les supports composites (7) présentent au moins chacun, sur deux côtés pratiquement opposés l'un a l'autre, un trou débouchant à travers lequel est introduit l'organe de liaison (9) en forme de fil.

3. Dispositif selon l'une des revendications 1 ou 2, caractérisé en ce que les supports composites (7) présentent une forme ergonomique.

4. Dispositif selon la revendication 3, caractérisé en ce que les supports composites (7) comportent des formes crochues (15).

5. Dispositif selon la revendication 3, caractérisé en ce que les supports composites (7) comportent des ouvertures (16, 17) pour leur remplissage et pour le durcissement du matériau composite.

6. Dispositif selon l'une des revendications 1 à 3, caractérisé en ce que l'organe de liaison (9) en forme de fil est réalisé en acier inoxydable ou en un matériau biocompatible pouvant se déformer de façon élastique et les supports composites (7) sont réalisés en matière plastique pouvant se déformer de façon plastique.
